# EUROPEAN PATENT APPLICATION

(11) **EP 4 059 424 A1**
(43) Date of publication of application: **21.09.2022**
(21) Application number: 21305346.5
(22) Date of filing: 20.03.2021
(51) Int. Cl.: A61B 5/145, A61B 5/00, A61B 5/16, A61B 5/021, A61B 5/024, A61B 5/053, G16H 50/20, A61B 5/0538, A61B 5/01, A61B 5/08, A61B 5/11, A61B 5/12

(54) **SYSTEM FOR MONITORING GASTRO-INTESTINAL DISORDERS**

(71) Applicant: Fondation de Coopération Scientifique, 67000 Strasbourg (FR)
(72) Inventor: MUTET, Bruno, 67500 HAGUENAU (FR); SWANSTROM, Lee, PORTLAND, OREGON 97210 (US); GONZALEZ CABRERA, Cristians Alejandro, Strasbourg, 67100 (FR)
(74) Representative: Plasseraud IP

(57) **Abstract**

The invention relates to a system for monitoring Gastro-intestinal disorders comprising:
A gastro-intestinal probe;
At least one sensor configured to measure a parameter of the patient;
A patient interface configured to receive inputs from the patient;
A processor configured to:
determine a first and second values corresponding to a given time interval:
wherein, if an input from the patient corresponds to the given time interval, said first value is determined based on a measurement of the parameter corresponding to the given time interval and on the input from the patient corresponding to the given time interval, and
wherein said second value is determined based on a measurement of gastro-esophageal content corresponding to the given time interval; and

determine a level of gastro-intestinal disorder based on the determined set of pairs of values.

## Description

The present invention generally relates to the domain of medical devices, and more specifically to systems for monitoring gastro-intestinal disorders.

Gastro-intestinal disorders have different forms and various levels of health consequence and impact on quality of life.

A large part of the diagnosis is done based on patient reported symptoms (PRS) and perceptions.

Diagnosis may also be based on systems relying on internal probes that are either swallowed or temporarily implanted against the gastro-intestinal tract wall of a patient to measure or capture information regarding the gastro-esophageal content. The gastro-esophageal content measurements (GECM) are sent and stored in a patient worn device.

More recently systems have been developed to base diagnosis on both, the GECM and the PRS, the PRS being inputted by the patient in its worn device. Such systems identify GECM events matching PRS events and use a known model to provide a disease severity scoring indication, for example, the symptom correlation score.

However, these technics lack efficiency. Indeed, the diagnosis relies mainly on the patient input of the PRSs which are relative to the patient perception and therefore highly subjective. In addition, the patient can often forget to input a PSR or simply delay its input causing imprecisions in the diagnosis. Moreover, when the patient activity is taken into account, it is a posteriori.

The present invention aims at improving the situation.

To that end, the invention relates to a system for monitoring gastro-intestinal disorders comprising:
A gastro-intestinal probe configured to perform gastro-esophageal content measurements of a patient;
At least one sensor configured to measure a parameter of the patient, said parameter being dependent to pain and/or stress of the patient;
A patient interface configured to receive inputs from the patient, said inputs corresponding to patient reported symptoms, PRS;

A processor configured to:
determine a set of pairs of values respectively corresponding to a set of different time intervals, each pair of values corresponding to a given time interval among the set of different time intervals comprises a first value and a second value,
wherein, if an input from the patient corresponds to the given time interval, said first value is determined based on a measurement of the parameter corresponding to the given time interval and on the input from the patient corresponding to the given time interval, and
wherein said second value is determined based on a measurement of gastro-esophageal content corresponding to the given time interval; and
determine a level of gastro-intestinal disorder based on the determined set of pairs of values.

It enables to enhance the accuracy of the diagnosis. Indeed, PRS experienced by the patient are somatic and therefore highly subjective. Thus, some patients describe high pain while others do not feel any pain for the same phenomena. By taking into consideration, during the time period measurements of the gastro-esophageal content are performed, a parameter dependent to pain and/or stress of the patient to determine the first value, enables to have an objective vu of the intensity of PRSs and to avoid miss interpreting a GECM event for which no PRS has been inputted by the patient while in fact experiencing some PRS.

Based on this objective first value, the level of gastro-intestinal disorder is therefore more relevant. This score may be obtained by correlating the events revealed by the first value and the second value which is based on measurement of gastro-esophageal content. Such correlation may be performed with existing techniques which combine PRS values and GECM values, for example, by replacing these values by respectively the first value and the second value. These techniques are for example, the symptom correlation score.

By gastro-intestinal probe it is understood any probe, especially internal probes that can measure through time a parameter of the gastro-esophageal content, for example, the pH of the gastro-esophageal content.

By parameter being dependent to pain and/or stress of the patient it is understood any parameter of the patient that varies when the patient experiences pain or stress, for example, increase of the heart rate, the temperature, the sweating, stress-related hormone levels, etc.

By inputs received from the patient it is understood any inputs, especially inputs related to the type and/or to the intensity of the PRS experienced by the patient.

By a set of different time intervals it is understood all-time division of a time period during which the gastro-esophageal content is measured. The time division can be predetermined, for example, a time partition in which each time interval is of the same duration. The time division can also be determined during the medical monitoring. For example, each gastro esophageal content measurement event, that is, each time a significant evolution of the parameter is measured starts a time interval which duration may be either the same for all the time interval, or different, for example, based on the intensity of the gastro esophageal content measurement event or until the next gastro esophageal content measurement event.

By parameter corresponding to a given time interval it is understood that the measurement of the parameter has been carried out in the given time interval.

By input from the patient corresponding to a given time interval it is understood that the input has been performed in the given time interval.

By measurement of gastro-esophageal content corresponding to the given time interval it is understood that the measurement of gastro-esophageal content has been carried out in the given time interval.

The first value is determined based on a measurement of the parameter, or more specifically on data representing the measurement of the parameter.

The determination of the level of gastro-intestinal disorder can be determined as a score related to the level of gastro-intestinal disorder, said score being determined based on the determined set of pairs of values.

According to an aspect of the invention the first value determined based on the measurement of the parameter and the input from the patient is determined by:
- weighting the measurement of the parameter according to the input from the patient, or
- weighting a value corresponding to the input from the patient according to the measurement of the parameter.

By weighting a value corresponding to the input from the patient according to the measurement of the parameter, it is understood that the event experienced by the patient and giving rise to the input is either valued or devalued according to the level of the parameter related to the pain and/or stress of the patient. Therefore, a PSR inputted while the level of the parameter related to pain and/or stress is low will be devalued and therefore less considered. In contrary, a PSR inputted while the level of the parameter related to pain and/or stress is high will be valued and therefore more considered.

By weighting a measurement of the parameter (for example, heartbeat) according to the PSR inputted, it is understood that the pain and/or stress experienced by the patient is either valued or devalued according to the PRS inputted. Therefore, the pain and/or stress experienced by the patient and from which results a PRS input indicating for example a low intensity PSR will be devalued and therefore less considered. In contrary, the pain and/or stress experienced by the patient and from which results a PRS input indicating for example a high intensity PSR will be valued and therefore more considered.

In both cases, the consideration of the parameter makes the first value more objective than if it was only based on the PRS inputted by the patient.

According to an aspect of the invention, if no input from the patient corresponds to the given time interval, the processor is further configured to determine said first value based on the measurement of the parameter corresponding to the given time interval.

In this case, no input from the patient is taken into account to determine the first value, which is based only on the measurement of the parameter. This enables to determine the first value even when the patient does not input any PRS, for example, when the patient is sleeping or when the patient forgets to input the PRS.

According to an aspect of the invention the second value is determined based on the measurement of gastro-esophageal content corresponding to the given time interval and on a patient activity information and/or a movement and posture information, said patient activity information and said movement and posture information corresponding to the given time interval.

This enables to take into consideration the activity of the patient when determining the second value. Indeed, evolution of the GECM may not be representative of Gastro-intestinal disorders, for example, doing sports may increase the GECM regardless of any Gastro-intestinal disorders. Therefore, the diagnosis is more accurate, for example, by not taking into account GECM related to the activity of the patient. This can be done by determining the second value by weighting the measurement of gastro-esophageal content according to the patient activity information and/or the movement and posture information.

By patient activity information corresponding to a given time interval it is understood that the information refers to the patient activity occurring during the given time interval. The patient activity is for example, having lunch, doing sport, sleeping, etc.

By movement and posture information corresponding to a given time interval it is understood that the information refers to movements and postures of the patient occurring during the given time interval. Movement and posture information can be determined based on any electronic sensor among an accelerometer sensor, a gyroscope sensor and a flexible angular sensor.

The patient activity information may be determined based on at least one among an output of a heart rate sensor, an output of a blood pressure sensor, an output of a temperature sensor, an output of a breathing rate sensor, an output of an oxygen saturation sensor, an output of an accelerometer sensor, an output of a gyroscope sensor, an output of a flexible angular sensor, an output of a clock, a geolocation information and an activity input given by the patient.

According to an aspect of the invention, the gastro-intestinal probe comprises at least one probe sensor among a pH sensor and a impedance sensor; and
wherein said gastro-esophageal content measurements are obtained based on the output of the at least one probe sensor.

Advantageously, the GECM can be obtained by a plurality of probe sensors arranged in array. This enables to determine the direction and speed of movement of the gastro-esophageal content in the gastro-intestinal tract.

According to an aspect of the invention, the sensor configured to measure the parameter is a sensor among a heart rate sensor, a blood pressure sensor, a temperature sensor, a breathing rate sensor, an oxygen saturation sensor, a lactate level sensor, a sodium level sensor, a uric acid level sensor, a potassium level sensor, a stress-related hormones sensor; and
wherein parameter measurements are obtained based on the output of the sensor configured to measure the parameter.

Indeed, the parameters measured by these different sensors are dependent to pain and/or stress of the patient.

According to an aspect of the invention, the system further comprises a receiving wireless communication unit configured to receive gastro-esophageal content measurements (more precisely data representing GECM) from the gastro-intestinal probe, and wherein the gastro-intestinal probe comprises a transmitting wireless communication unit configured to transmit gastro-esophageal content measurements.

Therefore, the data representing the GECM can be extracted from the probe to be analyzed while the probe is still in the gastro-intestinal tract.

In addition, the wireless communication unit can be included in a device, for example, a user equipment (smart phone, connected watch) or a patient worn device. Therefore, the patient can live normally which enables to monitor the patient during its dayto-day life, which is more relevant to establish an accurate diagnosis.

According to another aspect of the invention, the system comprises the gastro-intestinal probe, a patient worn device comprising at least one sensor configured to measure the parameter of the patient and a user equipment. The probe, the patient worn device and the user equipment have wireless communication capacity.

The user equipment's interface (for example the interface of a smartphone) can be used to collect data related to PRS while activity information can be retrieved from the different sensors and software already present on the smartphone. Such activity information may include meal times (start and end), meal content, sleeping time, time windows doing sport or sitting.

According to another aspect of the invention, the system associates the level of gastro-intestinal disorder to a specific gastro-intestinal disorder by correlating events revealed by the first value and the second value.

This enables to determine a specific disease among gastro-intestinal diseases. Indeed, each gastro-intestinal disease has a specific GECM and specific PRSs eventually depending on the activity of the patient (some specific gastro-intestinal disorder have symptoms (inducing PRS) which only occur when the patient practices certain activity). For example, symptoms associated to certain diseases only occur during a meal or during a time window after the end of the meal.

In addition, the system may determine another level of gastro-intestinal disorder related to another gastro-intestinal disorder by correlating events revealed by the first value and the second value .The determination of the another level is performed by the system in the same manner as to determine the level of gastro-intestinal disorder related to the gastro-intestinal disorder.

A second aspect of the invention concerns a method to monitor Gastro-intestinal disorders comprising:
- receiving data representing gastro-esophageal content measurements of a patient;
- receiving data representing parameter measurements, said parameter being dependent to pain and/or stress of the patient;
- receiving data representing inputs from the patient, said inputs corresponding to patient reported symptoms, PRS;
determining a set of pairs of values respectively corresponding to a set of different time intervals, each pair of values corresponding to a given time interval among the set of different time intervals comprises a first value and a second value,
wherein, if data representing inputs from the patient represents an input corresponding to the given time interval, said first value is determined based on a data representing a measurement of the parameter corresponding to the given time interval and on data representing the input from the patient corresponding to the given time interval, and
wherein said second value is determined based on data representing a measurement of gastro-esophageal content corresponding to the given time interval; and
- determining a level of gastro-intestinal disorder based on the determined set of pairs of values.

A third aspect of the invention concerns a computer program product comprising code instructions to perform the method as described previously when said instructions are run by a processor.

A fourth aspect of the invention concerns a comprising:
A communication interface configured to:
receive data representing gastro-esophageal content measurements of a patient;
receive data representing parameter measurements, said parameter being dependent to pain and/or stress of the patient;
receive data representing inputs from the patient, said inputs corresponding to patient reported symptoms, PRS;
the computer further comprising a processor configured to:
   determine a set of pairs of values respectively corresponding to a set of different time intervals, each pair of values corresponding to a given time interval among the set of different time intervals comprises a first value and a second value,
   wherein, if data representing inputs from the patient represents an input corresponding to the given time interval, said first value is determined based on a data representing a measurement of the parameter corresponding to the given time interval and on data representing the input from the patient corresponding to the given time interval, and
   wherein said second value is determined based on data representing a measurement of gastro-esophageal content corresponding to the given time interval; and
   determine a level of gastro-intestinal disorder based on the determined set of pairs of values.

The present invention is illustrated by way of example, and not by way of limitations, in the figures of the accompanying drawings, in which like reference numerals refer to similar elements and in which:
- Figure 1 illustrates a system according to an embodiment of the invention.
- Figure 2 illustrates a flowchart representing the implementation of a system according to the invention.

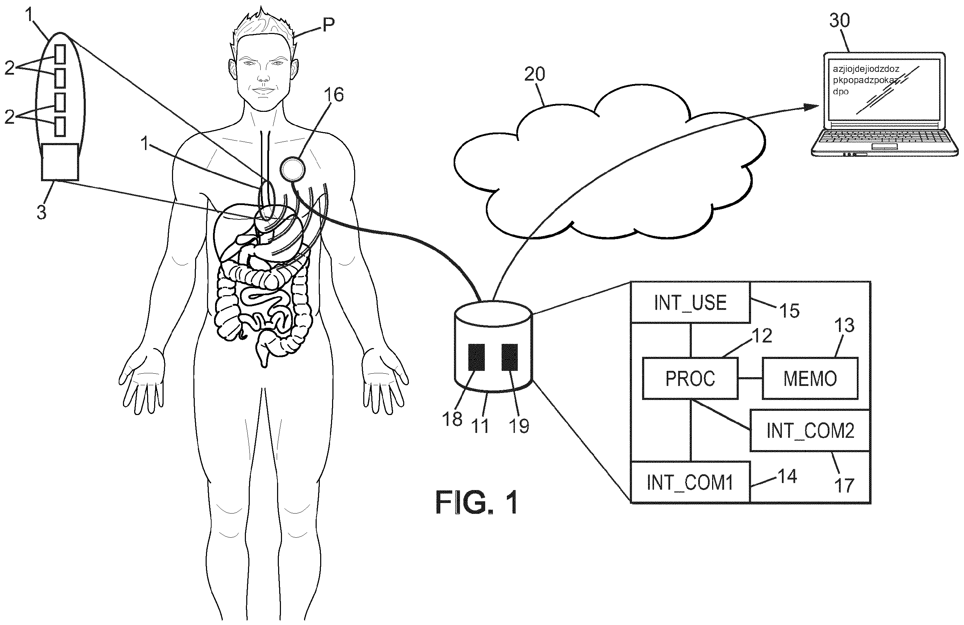

Referring to figure 1, there is shown a patient P with a gastro-intestinal probe 1 in its gastro-intestinal tract and a wearable device 11. The gastro-intestinal probe 1 comprises a plurality of probe sensors 2 arranged in array. The probe sensors 2 may be pH sensors and/or impedance sensors, these sensors output gastro-esophageal content measurements. The gastro-intestinal probe 1 comprises a wireless communication unit 3 which is configured to transmit the gastro-esophageal content measurements obtained via the probe sensors 2 to the wearable device 11.

The wearable device 11 (which could also be a user equipment) comprises a sensor 16 configured to measure a parameter of the patient, for example, the heartbeat of the patient P. More generally, the sensor 16 is of any type of sensor enabling to measure a parameter dependent to the pain and/or the stress of the patient P. Several sensors can be combined to track the evolution of several parameters dependent to the pain and/or the stress of the patient P. The sensor 16 may be a sensor among a heart rate sensor, a blood pressure sensor, a temperature sensor, a breathing rate sensor, an oxygen saturation sensor, a lactate level sensor, a sodium level sensor, a uric acid level sensor, a potassium level sensor and a stress-related hormones sensor.

The wearable device 11 comprises an activity sensor 18. The activity sensor 18 may be one among a heart rate sensor, a blood pressure sensor, a temperature sensor, a breathing rate sensor, an oxygen saturation sensor, an accelerometer sensor, a gyroscope sensor, a flexible angular sensor. Alternatively or in complement, the activity sensor 18 can be combined or replaced by a clock and/or a geolocation module.

The wearable device 11 comprises a movement and posture sensor 19. The movement and posture sensor 19 may be one among an accelerometer sensor, a gyroscope sensor and a flexible angular sensor. The activity sensor 18 and the movement and posture sensor 19 can be the same sensor.

The wearable device 11 comprises a patient interface 15 configured to receive inputs from the patient P corresponding to PRS and/or an activity input. The activity input refers to information inputted by the patient P and indicating an activity, for example, sleeping or having lunch. The wearable device 11 also comprises a wireless communication module 14, one processing module 12, a memory unit 13 and a communication module 17. The memory unit 13 comprises a non-volatile unit which retrieves the computer program and a volatile unit which retrieves the parameters used for establishing a score representative of a level of gastro-intestinal disorder, like a predefined length of the time intervals.

The wireless communication module 14 is configured to receive the gastro-esophageal content measurements transmitted by the wireless communication unit 3.

The wireless communication unit 3 and the wireless communication module 14 can use any wireless communication technologies and standard enabling to perform a wireless communication at a distance of about 1 meter, for example, LPWANs (Lora, Sigfox), cellular (3G, 4G, 5G), Mesh protocols (Zigbee), Bluetooth and BLE, Wifi or RFID.

The communication module 17 is configured to transmit data via a network 20 (for example, via internet using IP technology) to a computer 30, this computer being for example, the computer of the doctor which conducts the monitoring of the patient P to diagnosis a possible gastro-intestinal disorder.

The processing module 12 is configured to determine a set of time intervals, and for each time interval, to determine a first value and a second value. The processing module 12 is also configured to determine a score representative of a level of gastro-intestinal disorder. The processing module 12 is configured to determine an activity of the patient based on the output of the activity sensor 18 and/or on an activity input and thus determine activity information. The processing module 12 is configured to determine movement and posture information of the patient based on the output of the movement and posture sensor 19.

Thus, in the example of figure 1, the processor module 12 does all the computing required to determine the score representative of a level of gastro-intestinal disorder, however, the invention is not limited to such embodiment. Therefore, any or all of the computing required to determine the score can be done on the computer 30 of the doctor or on any other computer and/or server. For example, the communication module 17 can be configured to transmit the measurements received from the gastro-intestinal probe 1, the inputted PRS and the parameter measurements to the computer 30 of the doctor. Based on these received data, the computer 30 of the doctor can compute the first and second value and finally the score representative of a level of gastro-intestinal disorder.

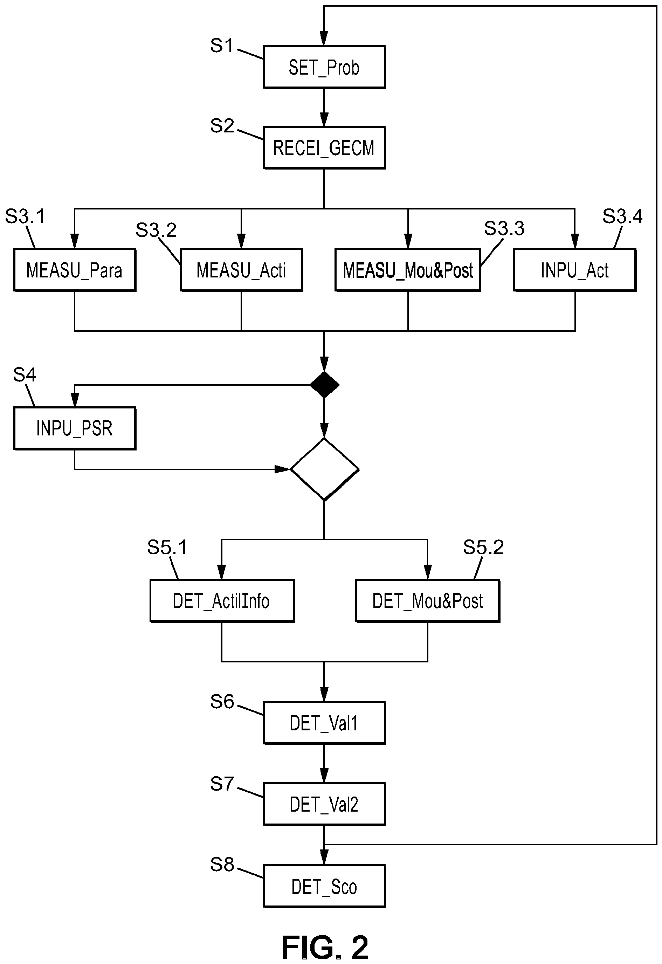

Referring to Figure 2, illustrates a flowchart representing the implementation of a system according to the invention.

At step S 1 the gastro-intestinal probe 1 is set in the gastro-intestinal tract of the patient P to be monitored for Gastro-intestinal disorders.

At step S2 the wearable device 11 receives GECMs from the probe 1.

When a specific GECM event occurs, the processing module 12 determines a time interval which begins at the time the GECM event is measured. The time interval lasts for a predetermined time T. As previously mentioned the time interval may be defined differently. For example, the duration of the time interval may depend on the intensity or the level of the GECM.

The GECM event is a significant variation and/or a significant level of GECM and/or a duration of GECMs above à level. For example, if the pH measured by the probe sensors 2 is under a certain level or if the pH measured decrease of a certain amount, then a GECM event is identified by the processing module 12.

At step S3.1, during the time interval, the wearable device 11 measures the level and tracks the evolution of the parameter through the sensor 16.

At step S3.2, during the time interval, the wearable device 11 receives signals from the activity sensor 18.

At step S3.3, during the time interval, the wearable device 11 receives signals from the movement and posture sensor 19.

At step S3.4, during the time interval, the wearable device 11 receives an activity input from the patient P through the patient interface 15.

At step 4, during the time interval, the wearable device 11 receives a PRS from the patient P through the patient interface 15. The PRS may combine a type of symptom (for example, heartburn, chest pain, pyrosis, belching, dysphagia, regurgitation or cough and a level of intensity of this symptom.

At step 5.1, the processing module 12 determines an activity information based on the activity input and on the signals received from the activity sensor 18. The activity information indicates the activity the patient is doing during the time interval. For example, if the activity sensor 18 is a temperature sensor and if the signal received from this temperature sensor corresponds to a low body temperature and that no activity input has been inputted, the activity information indicates that the patient is sleeping.

At step 5.2, the processing module 12 determines a movement and posture information based on the signals received from the movement and posture sensor 19. The movement and posture information indicates:
- the posture of the patient P during the time interval, for example, it indicates that the patient is lying down or bending over ;
- the movement of the patient P during the time interval, for example, it indicates that the patient is running, walking, doing sports or sleeping.

At step 6, the processing module 12 determines the first value.

If a PSR has been inputted during the time interval by the patient P, the first value is based on the PSR inputted and on the measures of the parameter dependent to the pain and/or the stress of the patient P.

The first value can be obtained by weighting a value corresponding to the inputted PSR according to the measurement of the parameter. For example, the first value is obtained by multiplying the level of intensity of the symptom experienced by the patient P by a weight coefficient. The weight coefficient is for example the maximum measured value of the parameter during the time interval normalized between 0 and 1. Therefore, if the measured level of the parameter during the time interval which follows the GECM event is low, the first value will be smaller than the inputted level of intensity of the symptom experienced by the patient P. In contrary, if the measured level of the parameter during the time interval which follows the GECM event is high, the first value will be greater than the inputted level of intensity of the symptom experienced by the patient P.

The first value can also be obtained by weighting the measurement of the parameter according to the inputted PSR. For example, the first value is obtained by multiplying the maximum measured value of the parameter during the time interval with a weight coefficient. The weight coefficient is for example the value of the inputted level of intensity of the symptom experienced by the patient P normalized between 0 and 1. Therefore, if the patient experience low intensity symptom during the time interval which follows the GECM event, the first value will be smaller than the maximum measured value of the parameter during the time interval. In contrary, if the patient experience high intensity symptom during the time interval which follows the GECM event, the first value will be greater than the maximum measured value of the parameter during the time interval.

If no PSR has been inputted during the time interval by the patient P, the first value is based on the measures of the parameter dependent to the pain and/or the stress of the patient P. For example, the first value is the maximum measured value of the parameter during the time interval.

In the previous the maximum measured value of the parameter is used to obtain the first value. However, any other relevant value related to the measures of the parameter can be used. For example, the average level of the parameter during the time interval can as well be used.

At step 7, the processing module 12 determines the second value based on the measurement of gastro-esophageal content measured at step S2. For example, to the GECM that has been detected as a GECM event.

If the activity information and the movement and posture information have been determined as it is the case in the steps S5.1 and S5.2, the second value is determined based on the GECM measured at step S2 and on these patient activity information and/or movement and posture information. For example, the processing module 12 obtains the second value by multiplying the value of the GECM measured at step S2 by a weight coefficient, the weight coefficient being based on the patient activity information and/or the movement and posture information. For example, a mapping can be pre-established assigning to each possible triplet of activities, postures and movements a weight coefficient. Therefore, based on the activities, postures and movements indicated by the patient activity information and the movement and posture information, the processing module 12 can retrieve and apply the relevant weight coefficient.

In the case, no activity information and movement and posture information have been determined, which for the sake of conciseness is not represented in figure 2, the second value is determined by the processing module 12 based only on the GECM measured at step S2. For example, the second value may be determined as the value of the GECM measured at step S2.

Several values of the GECM may be measured at step S2, that is, several measurements could have been performed during the time interval. This is especially the case with system performing high frequency measurement of the gastro esophageal content. In those cases the GECM used to compute the second value may be any combination of the GECM measured during the time interval, for example, the maximum GECM measured during the time interval, the average of the GECM measured during the time interval and the GECM that has been identified as the GECM event.

The steps S1 to S7 are iteratively performed to obtain a sequence of pairs of first and second value, each pair of values corresponding to its own time interval. Each iteration may involve a new time interval even though these time intervals may intersect.

At step S8, the processing module 12 determines a level of gastro-intestinal disorder. For example, by determining a score related to the level of gastro-intestinal disorder.

For example, for each pair of first and second values a third value is obtained, this third value being determined by multiplying the first value with a weighed coefficient. The weighed coefficient may be for example the second value (for example, normalized between 0 and 1). The sequence of third values obtained respectively with the sequence of pairs of first and second value is then compared to sequences of patient for which Gastro-intestinal disorders have been previously analyzed and for which diagnosis have been confirmed. The more the sequence of third values is similar to a sequence corresponding to a specific gastro-intestinal disorder and the more the score related to the level of gastro-intestinal disorder will be high.

More generally, the score may be determined based on known methods which may correlate classical PSR and GECM. In the case of the invention these methods are used with the first and second values instead.

## Claims

1. A system for monitoring Gastro-intestinal disorders comprising:
A gastro-intestinal probe configured to perform gastro-esophageal content measurements of a patient;
At least one sensor configured to measure a parameter of the patient, said parameter being dependent to pain and/or stress of the patient;
A patient interface configured to receive inputs from the patient, said inputs corresponding to patient reported symptoms, PRS;
A processor configured to:
determine a set of pairs of values respectively corresponding to a set of different time intervals, each pair of values corresponding to a given time interval among the set of different time intervals comprises a first value and a second value,
wherein, if an input from the patient corresponds to the given time interval, said first value is determined based on a measurement of the parameter corresponding to the given time interval and on the input from the patient corresponding to the given time interval, and
wherein said second value is determined based on a measurement of gastro-esophageal content corresponding to the given time interval; and
determine a level of gastro-intestinal disorder based on the determined set of pairs of values.

2. A system according to claim 1, wherein the first value determined based on the measurement of the parameter and the input from the patient is determined by:
- weighting the measurement of the parameter according to the input from the patient, or
- weighting a value corresponding to the input from the patient according to the measurement of the parameter.

3. A system according to claim 1 and 2, wherein, if no input from the patient corresponds to the given time interval, the processor is further configured to determine said first value based on the measurement of the parameter corresponding to the given time interval.

4. A system according to claims 1 to 3, wherein said second value is determined based on the measurement of gastro-esophageal content corresponding to the given time interval and on a patient activity information and/or a movement and posture information, said patient activity information and said movement and posture information corresponding to the given time interval.

5. A system according to claim 4, wherein the second value determined based on the measurement of gastro-esophageal content and based on the patient activity information and/or the movement and posture information is determined by weighting the measurement of gastro-esophageal content according to the patient activity information and/or the movement and posture information.

6. A system according to one of claims 4 and 5 comprising at least one electronic sensor among an accelerometer sensor, a gyroscope sensor and a flexible angular sensor, and wherein said movement and posture information is determined based on the output of the at least one electronic sensor.

7. A system according to one of claims 4 to 6, wherein the patient activity information being determined based on at least one among an output of a heart rate sensor, an output of a blood pressure sensor, an output of a temperature sensor, an output of a breathing rate sensor, an output of an oxygen saturation sensor, an output of an accelerometer sensor, an output of a gyroscope sensor, an output of a flexible angular sensor, an output of a clock, a geolocation information and an activity input given by the patient.

8. A system according to one of the previous claims wherein the gastro-intestinal probe comprises at least one probe sensor among a pH sensor and a impedance sensor; and
wherein said gastro-esophageal content measurements are obtained based on the output of the at least one probe sensor.

9. A system according to claim 8 wherein the gastro-intestinal probe comprises a plurality of probe sensors arranged in array,
wherein said gastro-esophageal content measurements are obtained based on the outputs of the plurality of probe sensors.

10. A system according to one of the previous claims wherein the sensor configured to measure the parameter is a sensor among a heart rate sensor, a blood pressure sensor, a temperature sensor, a breathing rate sensor, an oxygen saturation sensor, a lactate level sensor, a sodium level sensor, a uric acid level sensor, a potassium level sensor, a stress-related hormones sensor; and
wherein parameter measurements are obtained based on the output of the sensor configured to measure the parameter.

11. A system according to one of the previous claims further comprising a receiving wireless communication unit configured to receive gastro-esophageal content measurements from the gastro-intestinal probe, and wherein the gastro-intestinal probe comprises a transmitting wireless communication unit configured to transmit gastro-esophageal content measurements.

12. A system according to claim 11 further comprising a device, said device comprising the receiving wireless communication unit and/or the patient interface and said device being a user equipment or a patient worn device.

13. A system according to one of the previous claims further comprising associating the level of gastro-intestinal disorder to a first gastro-intestinal disorder and determining another level of gastro-intestinal disorder associated to a second gastro-intestinal disorder, said another level being determined based on the determined set of pairs of values.

14. A method to monitor Gastro-intestinal disorders comprising:
- receiving data representing gastro-esophageal content measurements of a patient;
- receiving data representing parameter measurements, said parameter being dependent to pain and/or stress of the patient;
- receiving data representing inputs from the patient, said inputs corresponding to patient reported symptoms, PRS;
- determining a set of pairs of values respectively corresponding to a set of different time intervals, each pair of values corresponding to a given time interval among the set of different time intervals comprises a first value and a second value,
wherein, if data representing inputs from the patient represents an input corresponding to the given time interval, said first value is determined based on a data representing a measurement of the parameter corresponding to the given time interval and on data representing the input from the patient corresponding to the given time interval, and
wherein said second value is determined based on data representing a measurement of gastro-esophageal content corresponding to the given time interval; and
- determining a level of gastro-intestinal disorder based on the determined set of pairs of values.

15. A computer program product comprising code instructions to perform the method according to claim 14, when said instructions are run by at least a processor.

16. A computer comprising:
A communication interface configured to:
receive data representing gastro-esophageal content measurements of a patient;
receive data representing parameter measurements, said parameter being dependent to pain and/or stress of the patient;
receive data representing inputs from the patient, said inputs corresponding to patient reported symptoms, PRS;
the computer further comprising a processor configured to:
determine a set of pairs of values respectively corresponding to a set of different time intervals, each pair of values corresponding to a given time interval among the set of different time intervals comprises a first value and a second value,
wherein, if data representing inputs from the patient represents an input corresponding to the given time interval, said first value is determined based on a data representing a measurement of the parameter corresponding to the given time interval and on data representing the input from the patient corresponding to the given time interval, and
wherein said second value is determined based on data representing a measurement of gastro-esophageal content corresponding to the given time interval; and
determine a level of gastro-intestinal disorder based on the determined set of pairs of values.
